# EUROPÄISCHE PATENTSCHRIFT

(11) **EP 1 083 911 B1**
(45) Veröffentlichungstag und Bekanntmachung des Hinweises auf die Patenterteilung: **09.03.2005**
(21) Anmeldenummer: 99938172.6
(22) Anmeldetag: 08.06.1999
(51) Int. Cl.: A61K 35/76, A61P 35/00

(54) **VERWENDUNG VON ADENOASSOZIIERTEN VIREN ZUR SENKUNG DER RADIO- ODER CHEMOTHERAPIEINDUZIERTEN RESISTENZ BEI KREBSPATIENTEN**
USE OF ADENO-ASSOCIATED VIRUSES FOR DECREASING THE RADIOTHERAPY-INDUCED OR CHEMOTHERAPY-INDUCED RESISTANCE IN CANCER PATIENTS
UTILISATION DE VIRUS ASSOCIES AUX ADENOVIRUS POUR ABAISSER LA RESISTANCE INDUITE PAR RADIOTHERAPIE OU CHIMIOTHERAPIE CHEZ DES PATIENTS ATTEINTS D'UN CANCER

(30) Priorität: 08.06.1998 DE 19825620
(43) Veröffentlichungstag der Anmeldung: 21.03.2001
(73) Patentinhaber: Deutsches Krebsforschungszentrum Stiftung des öffentlichen Rechts, 69120 Heidelberg (DE)
(72) Erfinder: VON KNEBEL-DOEBERITZ, Magnus, D-69118 Heidelberg (DE); KLEIN-BAUERNSCHMITT, Petra, D-69118 Heidelberg (DE); ZUR HAUSEN, Harald, D-69483 Waldmichelbach (DE); SCHLEHOFER, Jörg, D-69181 Leimen (DE)
(74) Vertreter: Schüssler, Andrea, Dr.
(86) Internationale Anmeldenummer: PCT/DE1999/001711
(87) Internationale Veröffentlichungsnummer: WO 1999/064024

(56) Entgegenhaltungen:
- WO-A-96/18737
- KLEIN-BAUERNSCHMITT P ET AL: "Improved efficacy of chemotherapy by parvovirus-mediated sensitisation of human tumour cells." EUROPEAN JOURNAL OF CANCER, (1996 SEP) 32A (10) 1774-80. , XP000867207
- HILLGENBERG M ET AL: "Enhanced sensitivity of small cell lung cancer cell lines to cisplatin and etoposide after infection with adeno-associated virus type 2." EUROPEAN JOURNAL OF CANCER, (1999 JAN) 35 (1) 106-10. , XP000867208

## Beschreibung

Die Erfindung betrifft die Verwendung von adenoassoziierten Viren zur Herstellung eines Medikaments zur Senkung der radio- oder chemotherapieinduzierten Resistenz bei Patienten, die an einem durch Radio- oder Chemotherapie zu behandelnden rezidivierenden Krebs leiden.

Die Therapie maligner Erkrankungen erfolgte bisher neben der chirurgischen Entfernung von Tumoren durch Radio- und/oder Chemotherapie. Der Einsatz zytostatischer Agentien wird jedoch durch das Auftreten von Nebenwirkungen, insbesondere durch die Ausbildung von Resistenzen, limitiert. Bedingt durch diese Nebenwirkungen können Chemotherapeutika nur in begrenztem Umfang eingesetzt werden. So kann nicht mehr als eine vom Patienten tolerierbare Dosierung des Chemotherapeutikums eingesetzt werden, die aber in den meisten Fällen nur einen geringen kurativen Effekt erzielt.

Aus Klein-Bauernschnitt, European J. of Cancer, Vol. 32A, No.10, S. 1774-1780 (1996) ist bekannt, daß die Kombination von AAV mit einem Chimiotherapeutikum die wachstumshemmende Wirkung des Chimiotherapeutikums auf Tumorzellen verbessert.

Der vorliegenden Erfindung liegt daher die Aufgabe zugrunde, das Problem der durch Radio- oder Chemotherapie induzierten Resistenz zu vermindern bzw. abzuschwächen, Dadurch soll die Überlebensrate von die an einem rezidivierenden Krebs leiden, patienten, nach einer Radio- oder Chemotherapie verbessert werden. Ferner sollen die Folgedosen bei einer Radio- oder Chemotherapie gesenkt werden können. Weiterhin soll die Ansprechbarkeit von Rezidiv- Tumorzellen auf Radio- oder Chemotherapie verbessert werden.

Diese Aufgabe wird durch die Gegenstände der Patentansprüche gelöst.

Erfindungsgemäß kann durch die Verwendung von adenoassoziierten Viren die Ausbildung einer radio- oder chemotherapieinduzierten Resistenz bei Patienten, die an einem durch Radio- oder Chemotherapie zu behandelnden rezidivierenden Krebs leiden, gesenkt werden.

Überraschenderweise wurde gefunden, daß nach einer Behandlung mit humanen apathogenen adenoassoziierten Viren (AAV) Rezidiv-Tumorzellen besser auf eine nachfolgende chemo- oder radiotherapeutische Maßnahme ansprechen.

Das AAV-Virus ist ein humanes Parvovirus, das eine Koinfektion mit einem Helfervirus benötigt, damit eine produktive Infektion stattfinden kann. AAV infiziert Menschen in früher Kindheit und gilt als apathogen, da keine menschliche Krankheit mit der AAV-Infektion verbunden werden konnte (Adv. in Vir. Res. 1987, 32, 43-306).

Eine Infektion mit AAV in Kombination mit chemo- oder radiotherapeutischen Maßnahmen steigert nach Erkenntnis der Erfinder die Effizienz der herkömmlichen Therapie und vermindert auftretende Resistenzen, so daß eine weitere Therapie erfolgversprechender als bisher durchgeführt werden kann. Erfindungsgemäß können beliebige AAV-Viren verwendet werden. Bevorzugt wird das AAV-2-Virus verwendet.

In Tierversuchen mit immundefizienten Nacktmäusen, denen subkutan kleinzellige Bronchialkarzinomzellen implantiert wurden, konnte gezeigt werden, daß eine AAV-Infektion, die gleichzeitig mit einer chemotherapeutischen Behandlung vorgenommen wurde, zu einem schnelleren Rückgang der Tumoren führte. In beiden Gruppen zeigten sich nach kurzer Zeit Rezidive. Diese sprachen jedoch in der AAV-infizierten chemotherapierten Gruppe auf eine erneute Chemotherapie besser an als in der nur chemotherapierten Gruppe. Daraus läßt sich erkennen, daß eine Infektion mit AAV die Ausbildung von Resistenzen vermindert oder sogar vermeidet.

Die erfindungsgemäße Verwendung der AAV-Viren kann vor, gleichzeitig oder nach der chemo- oder radiotherapeutischen Behandlung erfolgen. Sie erfolgt jedoch bevorzugt nach einem ersten chemo- oder radiotherapeutischen Behandlungszyklus.

Insbesondere bei Tumorarten, die per se schlecht auf eine chemo- oder radiotherapeutische Behandlung ansprechen, kann es angezeigt sein, die Behandlung vor der oder begleitend zu der Chemo-/Radiotherapie durchzuführen, um die Effizienz der Behandlung zu erhöhen. Dadurch können die Behandlungsdosen gesenkt werden, wodurch die Nebenwirkungen vermindert werden können.

Die Radio- oder Chemotherapie kann eine beliebige Radio- oder Chemotherapie sein, die dem zu behandelnden Krebs angepaßt ist. Solche Therapien sind seit Jahren bekannt und neben der chirurgischen Entfernung des Tumors die etablierte Methode, um Krebserkrankungen zu heilen bzw. die Lebenserwartung eines Patienten um einige Zeit zu erhöhen. Dem Fachmann sind deshalb die Maßnahmen einer Radio- oder Chemotherapie bestens bekannt.

Die erfindungsgemäße Verwendung der AAV-Viren kann auf beliebige Krebsarten angewendet werden, wobei beste Erfolge bei Colon-, Pankreas- und Hirntumoren (insbesondere Glioblastom) zu erwarten sind. Bevorzugt ist das kleinzellige Lungenkarzinom (SCLC) damit behandelbar.

Die erfindungsgemäß hergestellten Medikamente können intravenös, per infusionem, intratumoral, oral (auch per inhalationem) oder kutan appliziert werden. Dabei wird das Virus in einem geeigneten, dem Verabreichungsweg angepaßten Präparat formuliert. So ist es bevorzugt für eine intravenöse (auch als Infusion) und intratumorale Verabreichung das Virus in einer physiologischen Kochsalzlösung, Ringerlösung oder PBS-Lösung (Phosphatgepufferte Salzlösung), für eine kutane Verabreichung in Form einer Salbe, Suspension oder Gel, für eine orale Verabreichung in Form von Aerosolspray bereitzustellen.

Die verwendete Virusdosis beträgt abhängig vom Körpergewicht des Patienten 10⁹-10¹⁰ AAV-Partikel/kg KG.

Erfindungsgemäß kann wird auch eine pharmazeutische Zusammensetzung, verwendet werden, die neben dem Chemotherapeutikum (zytostatikum) adeno-assoziierte Viren, insbesondere AAV-2, enthält. Als Chemotherapeutikum sind alle bisher in der Tumortherapie gebräuchlichen Chemotherapeutika (Zytostatika) einzeln oder in Kombination einsetzbar, beispielsweise Cis-Platin, Etoposid, Methothrexat, Doxorubicin, Cyclophosphamid, Trofosfamid, Busulfan, Cytarabin, Fluoruracil, Mercaptopurin, Vinblastinsulfat, Vincristinsulfat, Bleomycinsulfat oder Mitomycin. So ist es bevorzugt für eine intravenöse (auch per infusionem) und intratumorale Verabreichung eine Injektionslösung, für eine kutane Verabreichung in Form einer Salbe, für eine orale Verabreichung in Form von Aerosolspray bereitzustellen. Als Basis für die Bereitung der Infusionslösung eignen sich jeweils in reiner Form oder als Mischung physiologische Kochsalzlösung, Ringerlösung oder PBS. Die Menge an AAV richtet sich nach dem Gewicht des Patienten und beträgt 10⁹-10¹⁰ Partikel/kg KG. In der pharmazeutischen Zusammensetzung ist diese in einer Menge für ein mittleres Körpergewicht von 70 kg enthalten. Die genaue Dosierung der pharmazeutischen Zusammensetzung wird vom Arzt festgelegt und ist abhängig vom Geschlecht und Gewicht des Patienten, Schwere der Krankheit, Art der Verabreichung und geplanter Dauer der Verabreichung. Die Zusammensetzung kann auch übliche Hilfsstoffe enthalten. Als Hilfsstoffe können die üblichen, wie Arzneimittelträger, Bindemittel, Sprengmittel, Gleitmittel, Lösungsmittel, Lösungsvermittler, Freigabe-Beschleuniger, Freigabe-Verzögerer, Emulgatoren, Stabilisatoren, Färbemittel oder Geschmackskorrigienzien verwendet werden.

Die Erfindung wird anhand der beigefügten Figuren näher erläutert.

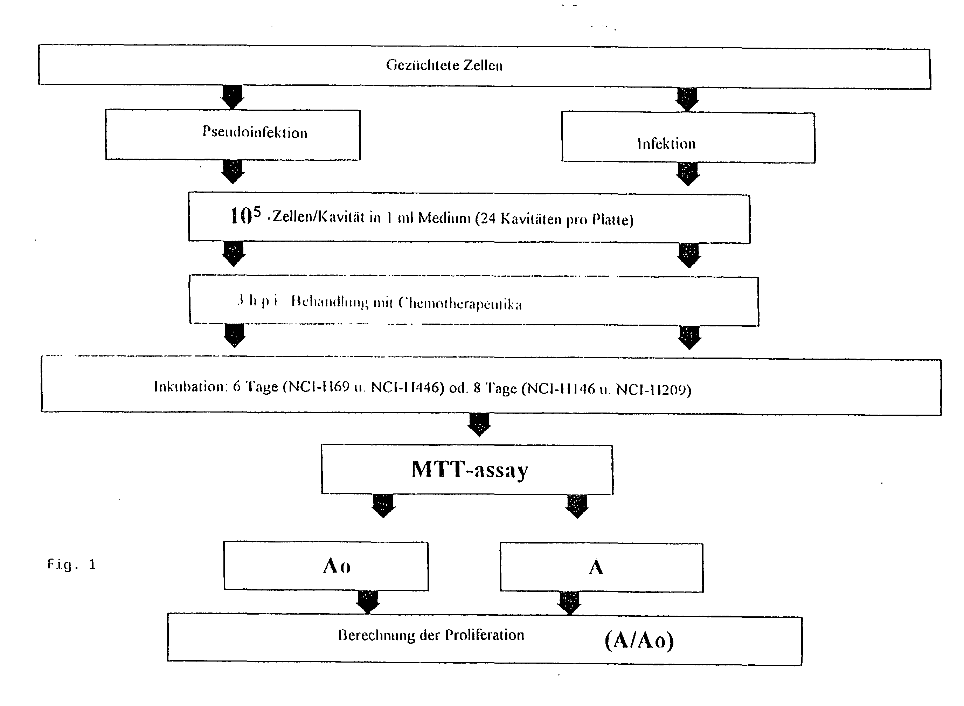

Figur 1 zeigt eine schematische Darstellung des zur Bestimmung von AAV-2 vermittelter Arzneimittel-sensibilisierung verwendeten Protokolls. Die Proliferation der SCLC-Zellinien nach der Infektion und/oder Arzneimittelbehandlung wurde mit dem MTT-Assay (J. Immunol. Methods 1983, 56, S. 55-63) bestimmt. Die relative Proliferation (A/Ao) wurde durch das Verhältnis der Absorption von AAV-2-infizierten und/oder mit dem Arzneimittel behandelten Zellen (A) zu der Absorption von pseudoinfizierten, unbehandelten Zellen (Ao) berechnet.

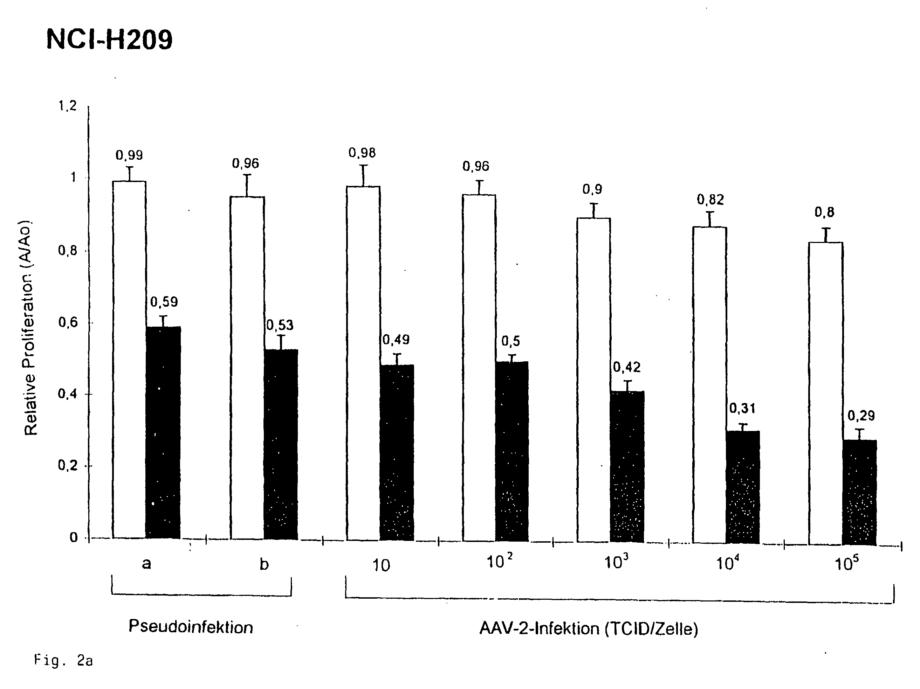

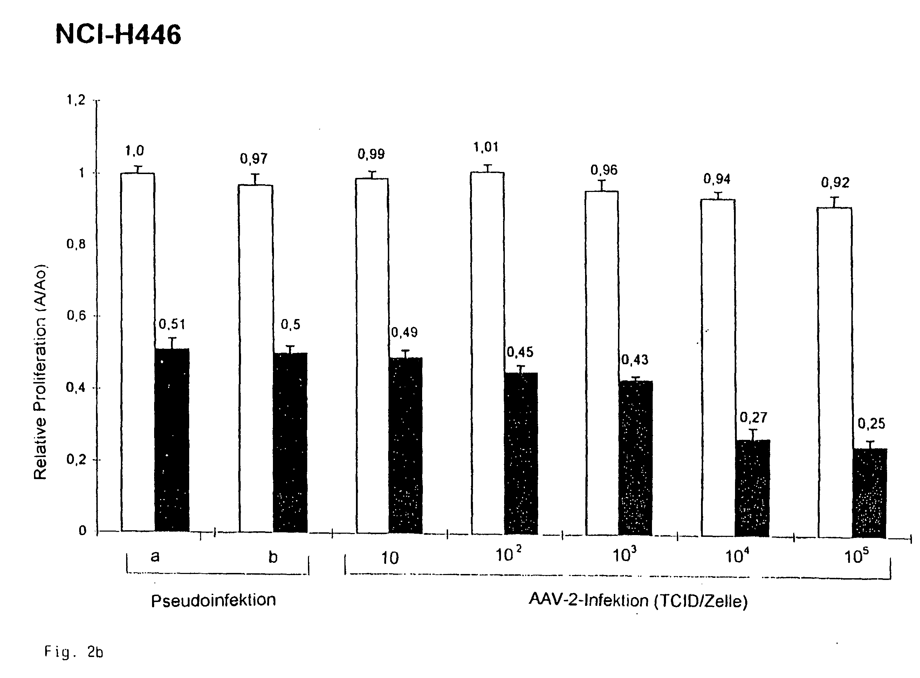

Die Figuren 2a+b zeigen die AAV-vermittelte Sensibilisierung der SCLC-Zellinien gegenüber Cis-Platin, die relative Proliferation (A/Ao) der SCLC-Zellinien, NCI-H209 (Figur 2a) und NCI-H446 (Figur 2b) nach Pseudoinfektion (a: PBS allein; b: infiziert mit einem hitzeinaktivierten Gradienten von Ad2-infizierten Zellen) oder Infektion mit verschiedenen Multiplizitäten einer Infektion mit AAV-2 mit (graue Säulen) oder ohne (weiße Säulen) anschließende Behandlung mit IC50 von Cisplatin gemäß Tabelle 1 (TCID, in der Gewebekultur infektiöse Dosis).

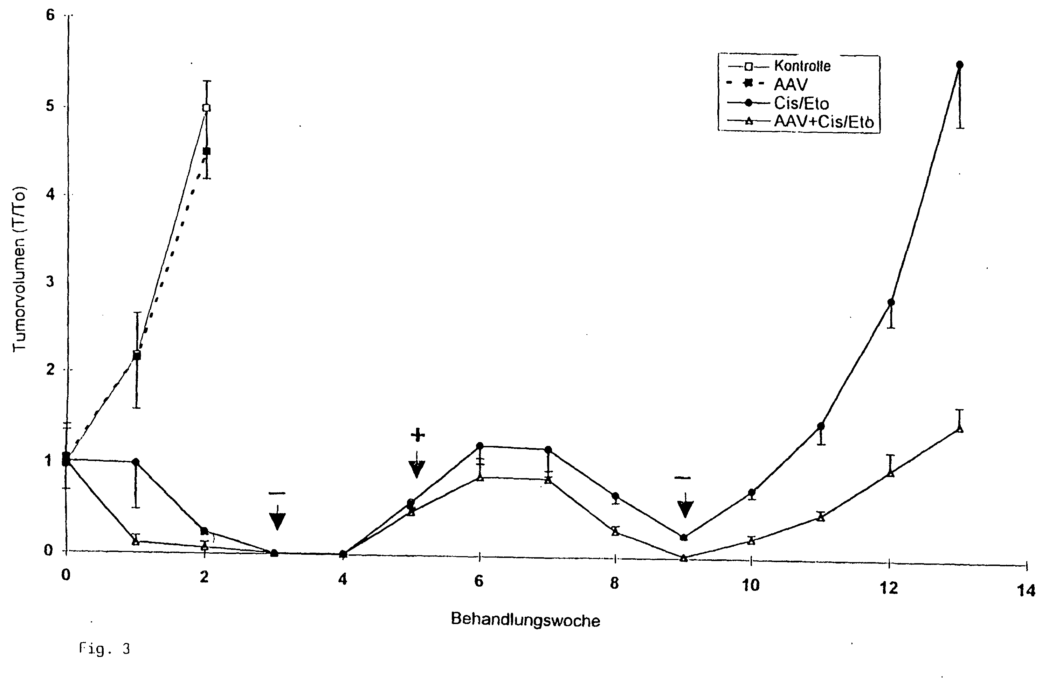

Die Figur 3 zeigt die AAV-vermittelte Sensibilisierung von von NCI-H209-Zellen abgeleiteten Tumoren in Nacktmäusen (5 Mäuse pro Gruppe). Die Dosis an Cis-Platin war 3 mg/kg Körpergewicht (wöchentliche Verabreichung). Die Etoposid-Dosis betrug 7,5 mg/kg Körpergewicht (dreimal wöchentlich verabreicht). AAV-2 wurde in einer MOI von 10⁸ TCID/Tier wöchentlich verabreicht. Pfeile zeigen die Änderung der Behandlungsmodalitäten an: - Unterbrechung der Arzneistoffbehandlung und AAV-2-Infektion; + Beginn der Behandlung und der Infektion.

Die Erfindung wird am Beispiel des kleinzelligen Lungenkarzinoms (SCLC) näher erläutert. Dieser Karzinomtyp ist im allgemeinen durch eine anfänglich effektive Chemotherapie und Remission des Tumors gekennzeichnet. Jedoch erleiden fast alle Patienten ein Rezidiv, das durch eine Resistenz der Tumorzellen gegenüber der zuerst angewendeten Chemotherapie entstanden ist (üblicherweise Cisplatin/Etoposid). Daher wurde in einem Modellsystem mit einer menschlichen kleinzelligen Lungenkarzinom-Zellinie getestet, ob eine Infektion mit AAV die zytotoxische Wirkung der Chemotherapeutika in der Zellkultur und in Tumoren bei immungestörten Mäusen verstärkt. Es wird gezeigt, daß die Infektion mit AAV signifikant die Wirksamkeit der chemotherapeutischen Behandlung an SCLC-Tumorzellen und -Tumoren erhöht.

### Beispiel 1

### Sensibilisierung von SCLC-Zellinien gegenüber Cis-Platin und Etoposid durch AAV-Infektion

Kleinzellige Lungenkrebszellinien (NCI-H69, NCI-H164, NCI-H209 und NCI-H446) (Cancer Res., 1980, 40, 3502-3507; Cancer Res., 1985, 45, 2913-2923) wurden in RPMI-1640-Medium (Eurobio, Raunheim, Deutschland) gezüchtet. HeLa-Zellen wurden in DMEN (Eurobio, Raunheim, Deutschland) gezüchtet. Alle Wachstumsmedien wurden mit Glutamin (Eurobio, 1%), Antibiotika (Penicillin und Streptomycin) und 10% hitzeinaktiviertem fötalem Kälberserum (PAA, Linz, Österreich) supplementiert. Die Kulturen wurden bei 37°C in einer Feuchtatmosphäre mit 5% CO₂ inkubiert und regelmäßig auf Mycoplasmenverunreinigungen getestet.

Das adeno-assoziierte Virus Typ 2 (AAV-2) wurde in HeLa-Zellen unter Verwendung von Adenovirus Typ 2 (Ad-2) als Helfer vermehrt. AAV wurde in einem Cäsium-chloridgradienten gereinigt und wie in J. Gen. Virol., 1994, 74, 2655-2662 beschrieben titriert. Die Adenovirus Typ 2 Inokula waren geklärte Überstände von Ad2-infizierten HeLa-Zellen.

Die SCLC-Zellen wurden in PBS suspendiert und mit gereinigtem AAV-2 in den angegebenen Multiplizitäten der Infektion (MOI) inkubiert. Nach 45 min bei 37°C wurde nichtgebundenes, absorbiertes Virus durch Waschen mit PBS entfernt und das Wachstumsmedium wurde ergänzt. Als Kontrollen (Pseudoinfektion) wurden entweder PBS oder die hitzeinaktivierte Fraktion (56°C, 30 min.) eines CsCl-Gradienten von mit Ad-2 infizierten Zellen alleine verwendet, wobei die Dichte (1,14 g/cm³) der AAV-2-haltigen Fraktion, die bei der AAV-2-Reinigung verwendet wurde, in den jeweiligen Experimenten angegeben ist. Das Volumen/Zell-Verhältnis bei diesen Experimenten war 50mal größer (5ml/10⁶ Zellen) als das, das für die AAV-Infektionen verwendet wurde.

Die AAV-2- oder pseudoinfizierten Zellen wurden mit Cis-Platin (Astra Medica, Frankfurt/Main, Deutschland) oder Etoposid (Bristol, München, Deutschland) oder beiden in einem Verhältnis [Cisplatin: Etoposide = 1:2,5] behandelt, wobei die Arzneistoffe aufgelöst in PBS dem Medium in der angegebenen Konzentration zugesetzt wurde.

Die Proliferation der SCLC-Zellen nach der Infektion mit AAV-2 und/oder Behandlung mit Chemotherapeutika wurde durch den modifizierten MTT-Test bestimmt (J. Immunol. Methods, 1983, 56, 55-63). Nach der Infektion oder Pseudoinfektion wurden die Zellen in Platten mit 24 Kavitäten in einer Dichte von 10⁵ Zellen/Kavität ausgebracht und mit den Chemotherapeutika in den angegebenen Konzentrationen behandelt. Nach sechs Tagen (NCI-H69, NCI-H446) bzw. acht Tagen (NCI-H146, NCI-H209) wurde der Kultur MTT ((3-(4,5-Dimethylthiazol-2-yl)-2,5-diphenyltetrazoliumbromid; Sigma, Deisenhofen, Deutschland) bis zu einer Endkonzentration von 0,5 mg/ml zugesetzt. Die Kulturen wurden dann 4 h bei 37°C inkubiert, um eine Reduktion von MTT zu blauem Formazan durch mitochondriale Dehydrogenasen (Arch. Biochem. Biophys., 1993, 303, 474-482) zu erlauben, was eine aktive Proliferation der Zellen anzeigt. Die Zellen wurden zentrifiguert, mit PBS gewaschen und Formazan wurde in Isopropanol solubilisiert. Die ausgefällten Proteine wurden durch Zentrifugation (1000 UpM, 15 min) pelletiert und 200 *µ*l-Proben des Überstands wurden gemessen, um die optische Dichte bei 540 nm (OD540) zu bestimmen, wobei die OD690 als Referenz und ein Titertek Multiskan plus MKII-Densitometer verwendet wurden (Lab Systems, Finnland).

Die relative Proliferation (A/Ao) wurde als das Verhältnis der in dem Überstand von AAV-2-infizierten und/oder mit Arzneistoff behandelten Zellen gemessene Absorption (A), verglichen mit der Absorption, die für den Überstand der pseudoinfizierten Zellen und unbehandelten Kontrollzellen gemessen wurde (Ao) definiert. Der IC50-Wert wurde als die Arzneistoff-Konzentrationen, die zu einer 50%-igen Hemmung der Proliferation führten, definiert.

Die mit AAV-2 infizierten Zellen (MOI-Werte wie angegeben) oder pseudoinfizierten Zellen wurden in Platten mit 24 Kavitäten ausgebracht und mit Cisplatin behandelt. Nach sechs bzw. acht Tagen wurde die relative Proliferation bestimmt. Zusätzlich wurden kinetische Studien durchgeführt, um die optimalen Behandlungsmodalitäten für SCLC-Zellen nach einer AAV-Infektion zu bestimmen. In diesen Untersuchungen wurde gezeigt, daß die AAV-vermittelte Sensibilisierung eine bis drei Stunden nach der Infektion maximal war. Bei dieser Untersuchung wurden die Chemotherapeutika drei Stunden nach der AAV-Infektion verabreicht. Um Effekte, die auf nach der Reinigung mit dem CsCl-Gradienten und der Ad2-Hitzeinaktivierung noch vorhandene Faktoren zurückzuführen sind, auszuschließen, wurde eine Kontrolle der Pseudoinfektion mit PBS zusätzlich zu der Pseudoinfektion mit der Fraktion des jeweiligen Gradienten eines Zellysats von mit Ad2 infizierten Zellen durchgeführt.

Es wurde die relative Proliferation von NCI-H209- und NCI-H446-SCLC-Zellen nach der Infektion mit verschiedenen Vielfachen der infektiösen Einheiten (MOI) von AAV-2 (10¹-10⁵ in der Gewebekultur infektiöse Dosis (TCID) pro Zelle) mit und ohne anschließende Behandlung mit Cisplatin bei den in Tabelle 1 aufgeführten IC50-Werten gemessen.

**Tabelle 1**

| Konzentration der Chemotherapeutika, die zu einer 50%-igen Hemmung der Proliferation (IC50) der SCLC-Zellinien führen | | | |
|---|---|---|---|
| | | | |
| NCI-H69 | 0,2 | 0,26 | 0,08 0,2 |
| NCI-H146 | 0,11 | 0,025 | 0,008/0,02 |
| NCI-H209 | 0,007 | 0,053 | 0,006/0,015 |
| NCI-H446 | 0,15 | 0,21 | 0,042/0,105 |

Wie in Tabelle 1 gezeigt, zeigten die SCLC-Zellinien NCI-H69 und NCI-H446 eine hohe intrinsische Resistenz gegenüber beiden Arzneimitteln, wobei die Empfindlichkeit gegenüber der Cisplatin/Etoposide-Behandlung niedriger war, wohingegen NCI-H146-Zellen gegenüber Etoposide hoch empfindlich waren und die NCI-H209-Zellen gegenüber beiden Arzneistoffen hoch empfindlich waren.

Wie aus Figur 2 (a+b) hervorgeht, führte die AAV-2-Infektion zu einer Abnahme der Proliferationsrate der mit Cisplatin behandelten Zellen bei einer MOI von 10³ bis 10⁴ TCID/Zelle. Die Infektion mit einer MOI von 10⁵ TCID/Zelle führte zu keiner weiteren Verstärkung. Keine signifikante Hemmung der Proliferation wurde nach der Infektion mit niedrigeren MOI-Werten von AAV-2 oder nach der Pseudoinfektion beobachtet, was eine spezifische Wirkung infolge der Infektion mit hoher MOI von AAV-2 anzeigt. Die relative Proliferation in AAV-2 infizierten (10³⁻⁵ AAV/Zelle) und mit Cisplatin behandelten (IC50) Zellen wurde auf 0,29 in NCI-H446-Zellen und auf 0,25 in NCI-H209-Zellen verglichen mit der relativen Proliferation in nur mit Cisplatin behandelten (IC50) Zellen (0,59 in NCI-H446-Zellen und 0,5 in NCI-H209-Zellen) erniedrigt.

### Beispiel 2

### Quantitative Bestimmung der AAV-2-vermittelten Arzneistoffsensibilisierung von SCLC-Zellinien

Um die Sensibilisierung von Zellen gegenüber Chemotherapeutika nach Infektion mit AAV-2 quantitativ zu bestimmen, wurden Dosis-Antwort-Kurven aufgestellt. Es wurde die relative Proliferation der Zellinien nach einer Pseudoinfektion (PBS) oder AAV-Infektion mit 10³ oder 10⁴ TCID/Zelle und anschließende Behandlung mit verschiedenen Konzentrationen von Cisplatin oder Etoposide oder eine Kombination beider Arzneimittel bestimmt (Tabelle 2).

**Tabelle 2**

| Sensibilisierungsfaktor von SCLC-Zellinien, die mit Cisplatin und/oder Etoposide behandelt und mit AAV infiziert wurden. | | |
|---|---|---|
| Zellinie | Chemotherapeutikum | Sensibilisierungsfaktor (A/Ao) *10⁴ Ip. AAV/Zelle |
| NCI-H69 | Cisplatin | 1,43 |
| | Etoposide | 1,30 |
| | Cisplatin/Etoposide | 1,45 |
| NCI-H146 | Cisplatin | 1,37 |
| | Etoposide | 1,38 |
| | Cisplatin/Etoposide | 1,33 |
| NCI-H209 | Cisplatin | 2,33 |
| | Etoposide | 2,12 |
| | Cisplatin/Etoposide | 2,00 |
| NCI-H446 | Cisplatin | 2,50 |
| | Etoposide | 3,00 |
| | Cisplatin/Etoposide | 3,00 |

| | | |
|---|---|---|
| *Die relative Proliferation (A/Ao) wurde durch das Verhältnis der Absorption von mit AAV-2-infizierten und/oder mit Arzneistoff behandelten (A) Zellen zu der Absorption der pseudoinfizierten, unbehandelten Kontrollen (Ao) berechnet | | |

Die Sensibilisierungsfaktoren (SF) wurden als das Verhältnis der IC50-Werte infizierter Zellen verglichen mit den IC50-Werten pseudoinfizierter Zellen definiert. Der Sensibilisierungsfaktor gibt den Faktor an, um den die Konzentration eines Chemotherapeutikums nach Infektion mit AAV-2 erniedrigt werden kann, um den gleichen Grad an Proliferationshemmung zu erhalten. Wie in Tabelle 2 zusammengefaßt war die Sensibilisierung durch AAV-2 in NCI-H69 und NCI-H146 mäßig (maximaler SF etwa 1,4 bei einer MOI von 10⁴ TCID/Zellen). Die Infektion von NCI-H209 oder NCI-H446 induzierte eine signifikantere MOI-abhängige Sensibilisierung (maximaler SF etwa 3 (NCI-H446) und 2,3 (NCI-H209) bei einer MOI von 10⁴ TCID/Zelle). Die AAV-2 vermittelte Sensibilisierung hing von dem verwendeten Chemotherapeutikum nicht ab.

### Beispiel 3

### AAV-2-vermittelte Arzneistoffsensibilisierung von MCI-H209-abgeleiteten Tumoren in Nacktmäusen

H209 ist eine Zellinie, die von einem Tumor abgeleitet ist, der vor der Züchtung nicht chemotherapeutisch behandelt wurde und nicht arzneimittelresistent ist.

Weibliche Nacktmäuse (CD1-nu/nu) von Iffa Credo (Brüssel, Belgien) wurden in Isolatoren gehalten und erhielten Wasser und Nahrung nach Belieben. Experimentell wachsende SCLC-Zellen (H209) wurden subcutan den sechs Wochen alten Mäusen (10⁷ Zellen in 100 *µ*l PBS pro Tier) in die Flanke injiziert. Fünf Monate nach der Inokulation der Zellen, als die Tumore ein Durchschnittsvolumen von 200 mm³ erreicht hatten, wurden die Tiere wöchentlich mit AAV-2 (intratumorale Injektion von 10⁸ in der Gewebekultur infektiösen Dosen (TCID)) infiziert und/oder mit Chemotherapeutica durch intraperitoneale Injektion von 3 mg/kg Körpergewicht Cis-Platin (wöchentlich) und 7,5 mg/kg Körpergewicht Etoposid (dreimal wöchentlich) behandelt. Einzelheiten des Beginns und des Endes der Behandlung sind in Figur 3 angegeben. In jeder Gruppe (Kontrolle, chemotherapeutische Behandlung, AAV2-Infektion, Behandlung + Infektion), wurden fünf Tiere aufgenommen. Die infizierten und nicht-infizierten Tiere wurden in separaten Isolatoren gehalten. Die Tumordurchmesser wurden wöchentlich gemessen und das Tumorvolumen wurde durch die Formel Tumorvolumen = ½ x Breite x Tiefe x Höhe bestimmt. Das relative Tumorvolumen (V/Vo) wurde für jedes Tier und jeden Zeitpunkt bestimmt (Verhältnis des Tumorvolumens [V] verglichen mit dem Tumor zu Beginn der Behandlung [Vo]).

Wie aus Figur 3 hervorgeht, führte die Behandlung mit Chemotherapeutika zu einer raschen Abnahme der Tumorvolumina und einer vollständigen Regression nach dreiwöchiger Behandlung. Die Kombination von Chemotherapie mit AAV2-Infektion führte zu einer rascheren Abnahme der Tumorvolumina verglichen mit Tieren, die nur eine Chemotherapie erhalten hatten, was eine Sensibilisierung der durch den Arzneistoff behandelten Tumorzellen durch AAV-2 anzeigt. Die Infektion mit AAV-2 alleine hatte keine signifikante Wirkung, und die Tumorvolumina nahmen in gleichem Umfang wie bei den unbehandelten Kontrollen zu. Die Behandlung wurde nach vollständiger Regression der Tumore unterbrochen und wurde bei einem Rezidiv wieder aufgenommen. Die Behandlung von Rezidiven war bei Tieren, die nur eine Arzneimittelbehandlung erhalten hatten weniger effektiv, verglichen mit der bei AAV-infizierten und chemotherapeutisch behandelten Tiere. Dies zeigt die Entwicklung einer Resistenz gegenüber der anfänglichen Behandlung bei mindestens der chemotherapeutisch behandelten Tiergruppe an. Die Rezidive in mit AAV-2 behandelten Tieren waren weiterhin gegenüber der Behandlung mit Cis-Platin und Etoposid empfindlich, aber die Tumorregression war langsamer verglichen mit der Regression der anfänglichen Tumore. In Woche 9 bildeten sich bei 3 von 5 mit AAV-2 infizierten Tieren die Tumore vollständig zurück, im Gegensatz zu den Tumoren bei Tieren, die nur mit Chemotherapeutika behandelt wurden, wobei eine vollständige Regression der Tumore nicht induziert wurde.

## Patentansprüche

1. Verwendung von adenoassoziierten Viren zur Herstellung eines Medikaments zur Senkung der radio- oder chemotherapieinduzierten Resistenz bei Patienten, die an einem durch Radio- oder Chemotherapie zu behandelnden rezidivierenden Krebs leiden.

2. Verwendung nach Anspruch 1, wobei die adenoassoziierten Viren in einer Dosis von 10⁹-10¹⁰ AAV-Partikel/kg KG eingesetzt werden.

3. Verwendung nach einem der Ansprüche 1 bis 2, wobei das adenoassoziierte Virus adenoassoziiertes Virus 2 (AAV-2) ist.

4. Verwendung nach einem der Ansprüche 1 bis 3, wobei der durch Radiooder Chemotherapie zu behandelnden Krebs ein Colon-, Pankreas- oder Hirntumor oder kleinzelliges Lungenkarzinom ist.

5. Verwendung nach einem der Ansprüche 1 bis 4, wobei die Verwendung intravenös, kutan, oral oder intratumoral erfolgt.

6. Verwendung nach einem der Ansprüche 1 bis 5, wobei die Verwendung vor, nach oder gleichzeitig zu einer Chemo- oder Radiotherapie erfolgt.

## Claims

1. Use of adeno-associated viruses for lowering the radiotherapy-induced or chemotherapy-induced resistance in patients who suffer from a recurring cancer which is to be treated by radiotherapy or chemotherapy.

2. Use according to claim 1, wherein the adeno-associated viruses are used in a dose of 10⁹-10¹⁰ AAV particles/kg body weight.

3. Use according to any of claims 1 to 2, wherein the adeno-associated virus is adeno-asscoiated virus 2 (AAV-2).

4. Use according to any of claims 1 to 3, wherein the cancer to be treated by radiotherapy or chemotherapy is a colon cancer, pancreatic carcinoma or brain tumor or small cell lung carcinoma.

5. Use according to any of claims 1 to 4, wherein the use is made intravenously, cutaneously, orally or intratumorally.

6. Use according to any of claims 1 to 5, wherein the use is made before, after or simultaneously with a chemotherapy or radiotherapy.

## Revendications

1. Utilisation de virus adéno-associés pour la préparation d'un médicament destiné à abaisser la résistance induite par radiothérapie ou chimiothérapie chez des patients atteints d'un cancer récidivant devant être traité par radiothérapie ou chimiothérapie.

2. Utilisation suivant la revendication 1, dans laquelle les virus adéno-associés interviennent à une dose de 10⁹-10¹⁰ particules de AAV/kg de poids corporel.

3. Utilisation suivant l'une des revendications 1 et 2, dans laquelle le virus adéno-associé est le virus adéno-associé 2 (AAV-2).

4. Utilisation suivant l'une des revendications 1 à 3, dans laquelle le cancer devant être traité par radiothérapie ou chimiothérapie est une tumeur du côlon, du pancréas ou du cerveau ou un cancer pulmonaire à petites cellules.

5. Utilisation suivant l'une des revendications 1 à 4, pratiquée par voie intraveineuse, cutanée, orale ou intratumorale.

6. Utilisation suivant l'une des revendications 1 à 5, pratiquée avant, après ou en même temps qu'une chimiothérapie ou une radiothérapie.
